(19) Europäisches Patentamt
European Patent Office
Office européen des brevets

(11) Veröffentlichungsnummer: **0 258 727**
**B1**

# (12) EUROPÄISCHE PATENTSCHRIFT

(45) Veröffentlichungstag der Patentschrift: **03.10.90**

(51) Int. Cl.[5]: **C07C 309/72,** G03F 7/022

(21) Anmeldenummer: **87111920.2**

(22) Anmeldetag: **18.08.87**

(54) **Verfahren zur Herstellung eines o-Naphtochinondiazidsulfonsäureesters.**

(30) Priorität: **27.08.86 DE 3629122**

(43) Veröffentlichungstag der Anmeldung:
**09.03.88 Patentblatt 88/10**

(45) Bekanntmachung des Hinweises auf die Patenterteilung:
**03.10.90 Patentblatt 90/40**

(84) Benannte Vertragsstaaten:
**DE FR GB**

(56) Entgegenhaltungen:
**US-A- 3 647 443**
**US-A- 4 397 937**

**Die Akte enthält technische Angaben, die nach dem Eingang der Anmeldung eingereicht wurden und die nicht in dieser Patentschrift enthalten sind.**

(73) Patentinhaber: **HOECHST AKTIENGESELLSCHAFT, Postfach 80 03 20, D-6230 Frankfurt am Main 80(DE)**

(72) Erfinder: **Erdmann, Fritz, Dr. Dipl.-Chem., Am Steinberg 3, D-6228 Eltville-Martinsthal(DE)**
Erfinder: **Staudt, Hans-Joachim, Adalbert-Stifter-Stasse 14, D-6204 Taunusstein-Hahn(DE)**
Erfinder: **Thamm, Horst, Dr.-Dipl.-Chem., In den Weingärten 26, D-6236 Eschborn 2(DE)**

**EP 0 258 727 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft die Herstellung eines o-Naphthochinondiazidsulfonsäureesters durch Veresterung eines o-Naphthochinondiazidsulfonsäurehalogenids, insbesondere -chlorids, mit einer ein- oder mehrwertigen phenolischen Verbindung in einem Lösungsmittel in Gegenwart einer basischen Komponente, Ausfällen des Esters und Trocknen. Die Erfindung betrifft auch die Verwendung des Esters in einem lichtempfindlichen Gemisch.

Es ist bekannt (DE-PS 865 860 entsprechend US-PS 3 046 120), daß Umsetzungsprodukte von o-Naphthochinondiazidsulfonsäurehalogeniden mit alkalilöslichen Phenolformaldehydharzen zu lichtempfindlichen Komponenten führen, die Grundlage von Kopierschichten sind.

Die Veresterungsreaktion zwischen dem o-Naphthochinondiazidsulfonsäurehalogenid und der phenolischen Verbindung wird dabei im allgemeinen in einem polaren organischen Lösemittel, wie beispielsweise Dioxan, Aceton, Methylethylketon oder einem Lösemittelgemisch in der Weise durchgeführt, daß als säurebindende, basische Komponente anorganische Basen wie Alkalicarbonate, -bicarbonate, Erdalkalicarbonate usw. in molaren Verhältnissen dem Reaktionsgemisch zugeführt werden.

Nach Abschluß der Reaktion können die Reaktionsprodukte in unterschiedlicher Weise, z.B. durch Ausfällen mit einem unpolaren Lösemittel oder durch Wasserzusatz aus dem Gemisch isoliert werden. Die gewonnenen Produkte enthalten verfahrensbedingt noch geringe Anteile der für die Kondensation verwendeten Metallionen und sind in dieser Form noch nicht uneingeschränkt brauchbar.

Es ist auch bekannt (DE-AS 2 044 868 entsprechend US-PS 3 647 443) die Reaktionen von o-Naphthochinondiazidsulfonylchlorid und phenolischen Komponenten in Lösungsmitteln, wie Pyridin, Picolin, Lutidin oder Triethylamin durchzuführen. Die Umsetzung kann dabei sowohl unter Normalbedingungen erfolgen oder auch bei verminderten oder erhöhten Temperaturen. Die Reaktionsprodukte können durch Neutralisation der Reaktionsmischung mit einer verdünnten Säure wie Salzsäure und Extrahieren mit einem Halogenkohlenwasserstofflösungsmittel z.B. Methylenchlorid isoliert werden.

Nachteilig an diesem Verfahren ist neben dem Einsatz von teilweise toxikologisch nicht unbedenklichen Basen der Anteil der verbleibenden Basenhydrochloride in dem Reaktionsprodukt.

Diese verbleibenden Restbestandteile können beim Aufarbeiten des Reaktionsproduktes zu Verfärbungen führen bzw. stören bei der Verarbeitung der Produkte zu lichtempfindlichen Systemen die empfindlichen Verarbeitungsanlagen durch Korrosion. Nachteilg ist auch der hohe technische Aufwand, der notwendig ist, um die verbliebenen Basenhydrochloride aus dem Reaktionsprodukt zu entfernen.

Die Ester der o-Naphthochinondiazidsulfonsäure und die aus ihnen herstellbaren positiv arbeitenden lichtempfindlichen Kopierschichten zum Beispiel für die Anwendung in der Leiterplattentechnologie und im besonderen für das Strukturieren von Siliziumoberflächen für die Anwendung in der Mikroelektronik müssen bei steigender Integrationsdichte den ständig wachsenden Anforderungen genügen. Eine der wichtigsten Anforderungen in dieser Hinsicht ist in letzter Zeit der Ausschluß von Metallionen in dem Reaktionsprodukt geworden.

Die Hauptanforderungen beziehen sich dabei auf das Fehlen von Ionen in den lichtempfindlichen Gemischen. insbesondere störend sind Metallionen wie beispielsweise Na, K, Mg, Mn, Fe, Bi, Sb, As, Cu, Cr, Ni, Zn, Se, weil sie entweder als sogenannte Dotierungselemente wirken oder durch Störungen im Kristallgitter der Siliziumoberflächen das spätere Ätzverhalten nachteilig beeinflußen.

Es war deshalb Aufgabe der Erfindung ein Verfahren zur Herstellung von o-Naphthochinondiazidsulfonsäureestern anzugeben, das die geschilderten Nachteile vermeidet und das den geforderten erhöhten Anforderungen der Mikroelektronik gerecht wird.

Die Lösung dieser Aufgabe geht aus von einem Verfahren der eingangs genannten Art, das dadurch gekennzeichnet ist, daß man die Veresterung in Gegenwart von Ammoniak, Ammoniumsalzen schwacher Säuren oder von primären oder sekundären aliphatischen Abkömmlingen des Ammoniaks mit 1 bis 3 Kohlenstoffatomen, wie Mono- oder Diethanolamin, bei einem pH-Wert im Bereich von etwa 1, 5 bis etwa 8, 5 und bei einer Temperatur im Bereich von etwa 15 bis etwa 40°C durchführt. Vorzugsweise führt man die Veresterun in Gegenwart von wässrigem Ammoniak oder von Ammoniumsalzen der Kohlen- oder Essigsäure durch. Dabei wird ein pH-Wert im Bereich von etwa 6 bis etwa 8 und eine Temperatur von etwa 20 bis etwa 35°C bevorzugt.

Hierdurch erreicht man, daß die störenden Begleitelemente der o-Naphthochinondiazidsulfonsäureester auf das notwendige Minimum reduziert oder gänzlich ausgeschlossen werden. Das Erfindungsgemäße Verfahren führt zu den gewünschten Produkten in hoher Reinheit und Ausbeute.

Das erfindungsgemäße Verfahren beinhaltet die Reaktion von o-Naphthochinondiazidsulfonsäurehalogenid, insbesondere dem entsprechenden Chlorid und der phenolischen Verbindung in der Weise, daß die Komponenten in polaren organischen Lösemitteln oder Lösungsmittelgemischen gelöst werden und unter sorgfältiger Kontrolle von Temperatur und pH-Wert in Gegenwart der erfindungsgemäßen basischen Komponente evtl. unter Zusatz von Wasser reagieren können.

Der Verfahrensablauf zu dem gewünschten o-Naphthochinondiazidsulfonsäureester ist insoweit überraschend, als aufgrund der Nucleophilie der dem Sulfonylhalogenid angebotenen Reaktionspartner erwartet werden konnte, daß die Hydrolysereaktion zur freien Sulfonsäure bzw. die Reaktion mit dem Amin zu dem entsprechenden Sulfonsäureamid bestimmend ist.

Ein besonderer Vorteil des erfindungsgemäßen Verfahrens ist neben der sehr guten Wasserlöslichkeit der entstehenden Basenhydrochloride und damit ihrer guten Auswaschbarkeit aus den Endprodukten, daß das im Anwendungsfall von Ammoniak entstehende Ammoniumchlorid thermisch nicht beständig ist und so bereits beim Trocknungsprozeß leicht und vollständig entfernt werden kann.

Die entstandenen Endprodukte erfüllen alle Anforderungen an lichtempfindliche Systeme für den Einsatz in der Mikroelektronik.

Zur Vermeidung von Verfärbungen und zur Unterdrückung von Nebenreaktionen ist es wichtig, den Ablauf der Kondensationsreaktion unter sorgfältiger Temperatur- und pH-Kontrolle ablaufen zu lassen.

Als Ammoniumsalze schwacher Säure kommen infrage Salze der Kohlensäure wie Ammoniumcarbonat oder Ammoniumbicarbonat, die durch die Reaktion zu leichtlöslichen und leichtflüchtigen Bestandteilen umgesetzt werden, die sich spätestens mit dem Trocknungsprozeß entfernen lassen, oder Salze der Essigsäure wie Ammoniumacetat.

Als aliphatische Abkömmlinge des Ammoniaks kommen primäre Amine mit 1 bis 3 Kohlenstoffatomen infrage, die weiter substituiert sein können. Ganz besonders bevorzugt sind Monoethanolamin und Diethanolamin.

Als o-Naphthochinondiazidsulfonsäurehalogenide werden in bekannter Weise die Chloride bevorzugt. Grundsätzlich sind auch die ebenfalls bekannten Bromide geeignet. Die Verbindungen leiten sich von dem Naphthochinon-(1, 2)-diazid, insbesondere dem -diazid-(2) ab. Die Verbindungen können im Molekül eine oder zwei Sulfonylhalogenidgruppen enthalten, die in 3-, 4-, 5-, 6-, 7- oder 8-Stellung des Naphthalinkerns stehen können. Von den Monosulfonsäuren werden zumeist die 4- und 5-Sulfonsäure, die letztere bevorzugt, verwendet. Bei Disulfonsäuren können die Sulfonsäuregruppen z. B. in 3, 5-; 3, 6-; 4, 6- oder 5, 7-Stellung stehen.

Als ein- oder mehrwertige phenolische Verbindung kommen vorzugsweise niedermolekulare Phenole in Betracht. Es können jedoch auch polymere Verbindungen mit phenolischen Hydroxygruppen oder auch Mischungen hieraus eingesetzt werden.

Als niedermolekulare Phenole kommen bevorzugt Verbindungen mit mindestens zwei Benzolkernen im Molekül in Betracht, die mindestens zwei phenolische Hydroxygruppen enthalten.

Eine bevorzugte Verbindungsgruppe sind Phenole der allgemeinen Formel

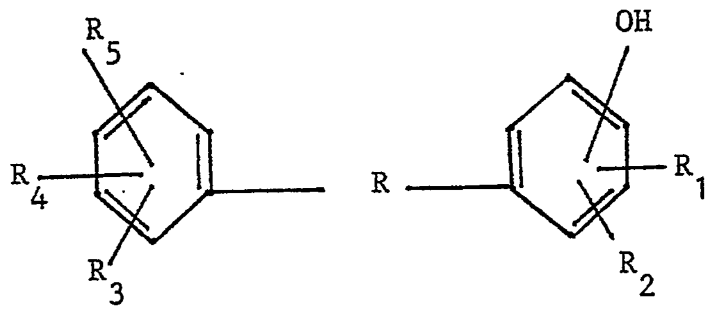

worin

R eine Einfachbindung oder eine der Gruppen CO, S, O, $SO_2$ und $CR_6R_7$, bevorzugt CO oder $CR_6R_7$,

$R_1$, $R_2$, $R_3$, $R_4$ und $R_5$ Wasserstoff- oder Halogenatome, Hydroxygruppen, Alkyl- oder Alkoxygruppen mit 1 bis 4 Kohlenstoffatomen,

$R_6$ und $R_7$ Wasserstoffatome oder Alkylgruppen mit 1 bis 4 Kohlenstoffatomen, bevorzugt Wasserstoffatome oder Methylgruppen, bedeuten

oder worin zwei der Reste

$R_3$, $R_4$ und $R_5$ und die Reste $R_1$ und $R_2$ jeweils zusammen einen aromatischen Ring bilden,

wobei stets mindestens einer der Reste $R_1$, $R_2$, $R_3$, $R_4$ und $R_5$ eine Hydroxygruppe bedeutet.

Beispiele für geeignete niedermolekulare phenolische Verbindungen sind 2,4-Dihydroxy-benzophenon, 2,3,4-Trihydroxy-benzophenon, 2,4,2',4'-Tetrahydroxy-diphenylsulfid, 2,2'-Dihydroxy-dinaphthylmethan, 4,4'-Dihydroxy-2,2'dimethyl-5, 5'-di-tert.-butyl-diphenylsulfid, 44'-Dihydroxy-diphenylsulfid, 4,6-Bis-(2,4-dihydroxy-phenylthio)resorcin, 2,4,2',4'-Tetrahydroxy-3,5,3',5'-tetrabromdiphenylsulfon, 2,4,2',4'-Tetrahydroxy-3,5,3',5'-tetrabrom-diphenyl oder 2,4-Dihydroxy-3,5-dibrom-benzophenon.

Als polymere Verbindungen mit phenolischen Hydroxygruppen kommen in erster Linie Kondensationsharze von Phenolen mit Carbonylverbindungen in Betracht. Hierbei werden als Reaktionspartner solche Kondensationsprodukte bevorzugt, die selbst als Bestandteile von o-Naphthochinondiazidschichten bekannt und gebräuchlich sind. Besonders bevorzugt werden deshalb Phenol-Formaldehyd- oder Kresol-Formaldehyd-Novolake. Die Verwendung der Veresterungsprodukte dieser Harze hat den Vorteil, daß ein Gehalt an unveresterter phenolischer Verbindung nicht stört, da diese ohnehin Bestandteil des Gemisches ist. Weitere geeignete Phenolharze sind Kondensationsprodukte von Pyrogallol und Aceton, wie sie in der GB-PS 1 113 759 beschrieben sind. Auch die in der DE-OS 2 847 878 beschriebenen Polykondensationsprodukte von Polyhydroxybenzophenonen und Formaldehyd können verwendet werden.

Im allgemeinen werden das o-Naphthochinondiazidsulfonsäurechlorid und die phenolische Verbindung in einem geeigneten Lösemittel bei Raumtemperatur gelöst und die für die Kondensation benötigte Base – vorzugsweise in Wasser gelöst – in dem Maße zudosiert, daß sich in dem Reaktionsgemisch eine Temperatur von 15 – 40°C einstellt und der pH-Wert die Grenzen von 1,5 – 8,5 vorzugsweise 6 – 8 nicht verläßt. Nach vollständigem Zudosieren der Base wird noch eine Zeit von etwa 0,5 bis 10 Minuten nachgerührt und das Gemisch aufgearbeitet.

Das entstandene Produkt wird abgetrennt, mehrfach mit Wasser gewaschen, getrocknet und kann in dieser Form direkt in lichtempfindlichen Gemischen eingearbeitet werden.

Als Bindemittel werden filmbildende Phenolharze mit einem Molekulargewicht von etwa 300 bis 5000 eingesetzt. Sie werden durch Kondensation von Phenol oder substituierten Phenolen mit Formaldehyd hergestellt. Geeignete substituierte Phenole sind Kresol, Xylenol, Butylphenol und ähnliche. Die besonders bevorzugten alkalilöslichen filmbildenden Phenolharze sind Phenol/Formaldehyd-Novolake, Kresol/Formaldehyd-Novolake und phenolmodifizierte Xylenol/Formaldehyd-Novolake. Die Mienge des Phenolharzes liegt im Bereich von etwa 50 bis 90 Gew.-%, bevorzugt 65 bis 85 Gew.-% der gesamten nichtflüchtigen Bestandteile.

Die erfindungsgemäßen Gemische können in an sich bekannter Weise noch Füllstoffe, Farbstoffe, Pigmente, photolytische Säurebildner, z.B. 1,2-Naphthochinondiazid-(2)4-sulfonsäurechlorid, und andere übliche Zusatz- und Hilfsstoffe enthalten.

Die Beschichtung des Trägermaterials erfolgt in an sich bekannter Weise, z.B. durch Aufsprühen oder Begießen.

Ein geeigneter Bereich des Schichtgewichtes auf Trockenbasis liegt zwischen 1,0 und 3,0 g/m$^2$.

Die Erfindung wird im einzelnen anhand der nachfolgenden Beispiele erläutert.

Beispiel 1:

Man löst

28,8 GT (Gewichtsteile) Naphthochinon-(1,2)-diazid(2)-5-sulfonylchlorid und

17,6 GT 2, 3, 4-Trihydroxy-benzophenon

in 161 GT Aceton und fügt unter pH- und Temperatur-Kontrolle

8,6 GT einer wässrigen 25 gewichtsprozentigen Ammoniaklösung

so der Acetonlösung der Reaktionskomponenten zu, daß die Temperatur zwischen etwa 15 bis 35°C und der pH-Wert zwischen 2 und 7,5 liegt.

Nach Zugabe rührt man noch 5 Minuten nach, säuert mit Salzsäure auf einen pH unter 2 an und gibt die Reaktionslösung in

1000 GT vollentsalztes Wasser (VE-Wasser)

Der entstehende Niederschlag wird in bekannter Weise über eine Nutsche abgesaugt. Durch sorgfältiges Waschen mit ca.

3000 GT vollentsalztem Wasser,

läßt sich das in Wasser sehr leicht lösliche Ammoniumchlorid vollständig auswaschen. Das abgezogene Waschwasser wird durch Leitfähigkeitsmessung kontrolliert, indem man den Niederschlag so lange wäscht, bis das ablaufende Wasser eine Leitfähigkeit von ≤ 10 µS aufweist. Nach Trocknen des Niederschlags erhält man

40 GT einer Mischung der Mono-, Bis- und Tris-ester obiger Ausgangsverbindungen, die in ionenfreien Mikroelektroniklacken direkt einsetzbar ist.

Beispiel 2:

Man löst

22 GT des Naphthochinon-(1,2)-diazid-(2)-5-sulfonylchlorids und

17,6 GT 2,3,4-Trihydroxy-benzophenon

in 161 GT Aceton

und fügt unter pH- und Temperaturkontrolle

66 GT Monoethanolamin

so der Acetonlösung der Reaktionskomponenten zu, daß die Temperatur zwischen etwa 15 und etwa 35°C und der pH-Wert zwischen etwa 2 und 7,5 liegt.

Nach Zufügen rührt man noch 5 Minuten nach, säuert mit Salzsäure an auf einen pH-Wert unter 2 und gibt die Reaktionslösung in

1600 GT VE-Wasser.

Der entstehende Niederschlag wird in bekannter Weise über eine Nutsche abgesaugt. Durch sorgfältiges Waschen mit

3000 GT VE-Wasser mit Überprüfung der elektrischen Leitfähigkeit des Waschwassers wie in Beispiel 1, läßt sich das sehr leicht lösliche Basenhydrochlorid vollständig auswaschen. Nach Trocknen des Niederschlags erhält man

33 GT einer Mischung der Mono-, Bis- und Tris-Ester obiger Ausgangsprodukte,

die für den Einsatz in metallionenfreien Elektroniklacken geeignet ist.

Beispiel 3:

Man löst

22 GT des Naphthochinon-(1,2)-diazid-(2)-5-sulfonylchlorids und

17,6 GT 2,3,4-Trihydroxy-benzophenon

in 167 GT Aceton
und fügt unter pH und Temperaturkontrolle
23,5 GT einer wässrigen 35 gewichtsprozentigen Ammonacetat-lösung
so der Acetonlösung der Reaktionskomponenten zu, daß die Temperatur zwischen etwa 15 bis etwa 35°C und der pHWert zwischen 2 und 7,5 liegt. Nach Zugabe rührt man noch 5 Minuten nach, säuert mit Salzsäure an auf einen pH-Wert unter 2 und überführt die Reaktionslösung in
1600 GT VE-Wasser.
Der entstehende Niederschlag wird abgenutscht. Durch sorgfältiges Waschen mit etwa
3000 GT VE-Wasser,
läßt sich das sehr leicht lösliche Ammoniumchlorid vollständig auswaschen. Nach Trocknen des Niederschlags erhält man
31 GT einer Mischung der Mono-, Bis- und Tris-ester obiger Ausgangsverbindungen,
die für den Einsatz in metallionenfreien Flüssigresists direkt einsetzbar ist.
Vergleichsbeispiel:
25 GT eines Polymerisats vom Novolak-Typ (Erweichungspunkt 112 – 119°C, Gehalt an phenolischen Gruppen 14 Gewichtsprozent) werden bei Raumtemperatur in
66 GT eines Gemisches aus Ethylglykolacetat, Essigsäurebutylester und Xylol (80:10:10) unter Rühren gelöst.
Der Lösung werden anschließend
7 GT einer Chinondiazidverbindung, hergestellt nach DE-PS 865 860, entsprechend US-PS 3 046 120, zugesetzt und das Gemisch wird bis zum vollständigen Lösen gerührt.
Nach entsprechender Aufarbeitung durch Filtration erhält man einen positiv arbeitenden, gebrauchsfertigen Photolack mit der folgenden Metallionenkonzentration:
Na+ 55ppm; Fe++/+++ 2,9 ppm; Ca++ 1,5 ppm,
wobei die Konzentrationen mit Hilfe der Atomabsorptionsspektroskopie gemessen wurde.
Beispiel 4:
25 GT eines Polymerisats vom Novolak-Typ wie im Vergleichsbeispiel werden bei Raumtemperatur in
68 GT eines wie im Vergleichsbeispiel beschriebenen Lösungsmittelgemischs gelöst.
Der Lösung werden anschließend
7 GT einer Naphthochinondiazidsulfonsäureestergemisch von Beispiel 1 zugesetzt. Das Gemisch wird bis zum vollständigen Lösen gerührt.
Nach entsprechender Aufarbeitung erhält man einen positiv arbeitenden, gebrauchsfertigen Photolack mit den folgenden Metallionenkonzentrationen:
Na+: unter 1 ppm; Fe++/+++ : unter 1 ppm;
K+, Mg+, Ca++, Cu++ und Mn++ jeweils unter 1 ppm.

**Patentansprüche**

1. Verfahren zur Herstellung eines o-Naphthochinondiazidsulfonsäureesters durch Veresterung eines o-Naphthochinondiazidsulfonsäurehalogenids mit einer ein- oder mehrwertigen phenolischen Verbindung in einem Lösungsmittel in Gegenwart einer basischen Komponente, Ausfällen des Esters und Trocknen, dadurch gekennzeichnet, daß man die Veresterung in Gegenwart von Ammoniak, Ammoniumsalzen schwacher Säuren oder von primären oder sekundären aliphatischen Abkömmlingen des Ammoniaks mit 1 bis 3 Kohlenstoffatomen, wie Monoethanolamin oder Diethanolamin, bei einem pH-Wert im Bereich zwischen etwa 1,5 und etwa 8,5 und bei einer Temperatur im Bereich zwischen etwa 15 und etwa 40°C durchführt.

2. Verfahren nach Anspruch 1, dadurch gekennzeichnet, daß man die Veresterung in Gegenwart von wässrigem Ammoniak durchführt.

3. Verfahren nach Anspruch 1, dadurch gekennzeichnet, daß man die Veresterung in Gegenwart von Ammoniumsalzen der Kohlen- oder Essigsäure durchführt.

4. Verfahren nach Anspruch 1, dadurch gekennzeichnet, daß man die Veresterung bei einem pH-Wert im Bereich von etwa 6 bis 8 durchführt.

5. Verfahren nach Ansprüchen 1 bis 4, dadurch gekennzeichnet, daß man die Veresterung bei einer Temperatur im Bereich von etwa 20 bis etwa 35°C durchführt.

**Claims**

1. Process for the preparation of an o-naphthoquinonediazide sulfonic acid ester by esterification of an o-naphthoquinonediazide sulfonic acid halide with a mono- or polyvalent phenolic compound, in a solvent in the presence of a basic component, followed by precipitation of the ester and drying, characterized in that the esterification is performed in the presence of ammonia, of ammonium salts of weak acids or of primary or secondary aliphatic derivatives of ammonia having 1 to 3 carbon atoms, such as monoethanolamine or diethanolamine, at a pH within the range from about 1.5 to about 8.5 and a temperature within the range from about 15°C to about 40°C.

2. Process as claimed in claim 1, characterized in that the esterification is performed in the presence of aqueous ammonia.

3. Process as claimed in claim 1, characterized in that the esterification is performed in the presence of ammonium salts of carbonic or acetic acid.

4. Process as claimed in claim 1, characterized in that the esterification is performed at a pH within the range from about 6 to about 8.

5. Process as claimed in claims 1 to 4, characterized in that the esterification is performed at a temperature within the range from about 20°C to about 35°C.

## Revendications

1. Procédé pour la préparation d'un ester d'acide o-naphtoquinonediazide-sulfonique par estérification d'un halogénure d'o-naphtoquinonediazide-sulfonyle par un composé mono- ou polyphénolique, dans un solvant en présence d'un composant basique, précipitation de l'ester et séchage, caractérisé en ce que l'on effectur l'estérification en présence d'ammoniac, de sels d'ammonium d'acides faibles ou de dérivés aliphatiques primaires ou secondaires de l'ammoniac, ayant de 1 à 3 atomes de carbone, tels que la monoéthanolamine ou la diéthanolamine, à un pH dans l'intervalle compris entre environ 1,5 et environ 8,5, et à une température dans la plage comprise entre environ 15 et environ 40°C.

2. Procédé selon la revendication 1, caractérisé en ce que l'on effectue l'estérification en présence d'ammoniac en solution aqueuse.

3. Procédé selon la revendication 1, caractérisé en ce que l'on effectue l'estérification en présence de sels d'ammonium de l'acide carboniue ou acétique.

4. Procédé selon la revendication 1, caractérisé en ce que l'on effectue l'estérification à un pH dans l'intervalle d'environ 6 à 8.

5. Procédé selon les revendications 1 à 4, caractérisé en ce que l'on effectue l'estérification à une température dans la plage d'environ 20 à environ 35°C.